# EUROPÄISCHE PATENTANMELDUNG

(11) **EP 0 553 448 A1**
(43) Veröffentlichungstag der Anmeldung: **04.08.1993**
(21) Anmeldenummer: 92120915.1
(22) Anmeldetag: 08.12.1992
(51) Int. Cl.: A61C 9/00, A61K 6/00, A61K 6/10, C09J 4/06

(54) **Oberflächenkonditionierter Abformlöffel**

(30) Priorität: 09.12.1991 DE 4140504; 27.08.1992 DE 4228538
(71) Anmelder: A. KETTENBACH GmbH & CO. KG., D-35713 Eschenburg (DE)
(72) Erfinder: Göbel,Roland,Dr., O-6900 Jena (DE); Musil,Rudolf, Prof.Dr., O-6900 Jena (DE); Welker,Dieter, O-6900 Jena (DE)
(74) Vertreter: Pfeiffer, Rolf-Gerd

(57) **Zusammenfassung**

Die Erfindung betrifft einen oberflächenkonditionierten Abformlöffel für die Dentaltechnik, der zur Anfertigung von Abdrücken eingesetzt wird. Die Aufgabe der Erfindung, der Oberfläche des Abformlöffels eine solche Konditionierung zu geben, die eine ganzflächige Haftung des Abformmaterials bei der Abdrucknahme und Verfestigung des Abformmaterials auch bei mehreren Abformungen unverändert gewährleistet wird erfindungsgemäß dadurch gelöst, daß der Abformlöffel zumindest im Bereich der Aufnahme des Abformmaterials mit einer Verbundschicht versehen ist, die aus einer verfestigten Phenolharz-Methacrylat-Dispersion besteht.

## Beschreibung

Die Erfindung betrifft einen oberflächenkonditionierten Abformlöffel, der zur Anfertigung naturgetreuer Modelle über ein Negativverfahren in der Zahnmedizin häufig zum Einsatz gelangt.

Die zur Abformung gebräuchlichsten Abformwerkstoffe sind Alginate und Elastomere. Sie werden aufgrund ihrer einfachen Handhabbarkeit, geringer Kosten sowie ihrer hohen Wiedergabegenauigkeit bevorzugt verwendet.

Der Abformlöffel, als unabdingbarer Träger des elastischen Abformmaterials, trägt neben dem Abformmaterial in ganz entscheidendem Maße zur Herstellung präziser Modelle bei.

Da beim Entfernen des Abdrucks vom Objekt durchaus Kräfte bis 20 kp auftreten können, werden stabile, biegefeste, anatomisch geformte Löffel aus Metall den ungebräuchlichen Plastelöffeln vorgezogen.

Beim Abbindevorgang der eingesetzten Abformmaterialien schrumpfen Alginate etwa um 2% und Elastomere um 0,4 bis 1,2%. Bei guter Haftung des Abformmaterials am Abformlöffel wird das anschließend zu erhaltene Modell durch die Materialkontraktion des Abformmaterials um dessen Schrumpfungsanteil minimal größer, was als günstig zu erachten ist. Ein fester Verbund von Abformmaterial und Abformlöffel ist für die Festlegung einer definierten Kontraktionsrichtung verantwortlich. Damit ist eine Steuerung der Modelldimension durch die Kombination von Abformund Modellmaterial möglich. Auch eine nur teilweise Ablösung des Abformmaterials vom Abformlöffel würde dagegen eine Kontraktion des Abformmaterials in Richtung seines Massezentrums zulassen und eine nicht mehr handhabbare Dimensionsänderung bewirken.

Seit Einführung der hydrokolloidalen bzw. elastomeren Abformmassen ist bekannt, daß der adhäsive Verbund dieser Massen mit der Abformlöffeloberfläche nicht ausreicht, um zu gewährleisten, daß sich das Abformmaterial während der Aushärtung, als auch bei der Abformung nicht von der Löffeloberfläche löst.

Es sind eine Reihe von Möglichkeiten vorgeschlagen worden, die Haftung des Abformmaterials zur Löffeloberfläche zu verbessern. So kann der Löffel vor der Abformung mit einem Haftlack bestrichen bzw. mit einer Haftfolie beklebt werden. Darüber hinaus kann der Löffel selbst perforiert bzw. an seiner Innenwand ein Kupferdraht angelötet sein (Rimlocklöffel). Alle diese Möglichkeiten garantieren jedoch keine gesicherte ganzflächige Haftung des Abformmaterials am Abformlöffel. Desweiteren ist es nachteilig, daß die haftverbessernden Maßnahmen vor jeder Abformung erneut ausgeführt werden müssen.

Es ist daher Aufgabe der Erfindung, der Oberfläche des Abformlöffels eine solche Konditionierung zu geben, die eine ganzflächige Haftung des Abformmaterials bei der Abdrucknahme und Verfestigung des Abformmaterials auch bei mehreren Abformungen unverändert gewährleistet.

Die Aufgabe wird durch die kennzeichnenden Mittel der Patentansprüche gelöst.

Erfindungsgemäß wird auf die Oberfläche des Abformlöffels eine Phenol-Formaldehyd-Dispersion aufgestrichen, die mit einem Anteil an olefinischen ungesättigten ein- bzw. mehrfunktionellen Methacrylatverbindungen versehen ist. Anschließend wird diese Schicht einer Aushärtung unterworfen. Bei den polymerisationsfähigen olefinischen ungesättigten Monomeren kann es sich auch um Alkoxysilane mit mindestens einer polymerisationsfähigen olefinischen ungesättigten Gruppe, wie Methacrylsäure-3-trimethoxysilylpropylester oder Vinyltrimethoxysilan handeln. Insbesondere setzt sich die erfindungsgemäße Dispersion, bezogen auf 100 ml Lösung aus 0,5 bis 5 g Phenol, 0,25 bis 2,5 g Formaldehyd, 0,05 bis 0,5 g Polyvinylformal bzw. Polyvinylbutyral, 1 bis 5 g dispergiertem Acrylat, 10 bis 80 ml destilliertem Wasser und 10 bis 80 ml Aceton bzw. einem anderen Lösungsmittel, wie z.B. Isopropanol zusammen.

Dieser Dispersion sind 2 bis 20 g einer oder mehrerer olefinischer ungesättigter ein- bzw. mehrfunktioneller Methacrylatverbindungen zugesetzt, so daß im Ergebnis eine erfindungsgemäße Phenolharz-Methacrylat-Dispersion entsteht. Diese Dispersion wird auf die Oberfläche des Abformlöffels aufgestrichen und einer Temperaturbehandlung zwischen 120 und 220 °C unterworfen.

Ein derart oberflächenkonditionierter Abformlöffel garantiert eine gegenüber dem Stand der Technik wesentlich verbesserte Haftung der Abformmaterialien, wie z.B. additionsvernetzten Silikonen von 50 bis 60 Ncm⁻². Als äußerst vorteilhaft erweist es sich dabei, daß mit der Oberflächenkonditionierung nicht nur ein einmaliger Verbund zum Abformmaterial erreichbar ist, sondern nach einer Sterilisation des Löffels z.B. durch einstündige Heißluftbehandlung bei 180°C oder einstündige Einlagerung in eine 70%-ige Alkohol-Wasser-Lösung, die Oberflächenaktivität erfindungsgemäßer Beschichtung erhalten bleibt und keine Haftverminderung zum Abformmaterial auftritt.

Durch die erfindungsgemäße dauerhafte aktive Beschichtung entfällt somit die zeitaufwendige Vorbehandlung, die bei der Anwendung von Additiven vor jeder Behandlung notwendig ist. Der erfindungsgemäß beschichtete Abformlöffel ist nach der Sterilisation sofort wieder einsetzbar. Es liegt im Rahmen der Erfindung, um eine möglichst große mechanische Oberfläche zu erhalten, den Abformlöffel vor Aufbringung der erfindungsgemäßen Schicht zunächst zu sandstrahlen und anschließend mit einer mikroporösen silikatischen Schicht mit einer Schichtdicke von 50 bis 100 µm zu überziehen. Diese Schicht wird vorteilhaft durch Flammhydrolyse einer Alkoxysilanverbindung aufgebracht und stellt das Haftbett für die erfindungsgemäße Konditionierungsschicht dar. Ebenso liegt es im Rahmen der Erfindung, die mit einer silikatischen Oberfläche versehene Metallschicht des Abformlöffels mit einer Silikonöl-Essigsäureethylester-Lösung, deren Silikonölanteil zwischen 5 bis 50% liegt, zu bestreichen und die so vorbehandelte Schicht einer ca. 15-minütigen Temperaturbehandlung zwischen 180 - 240°C zu unterziehen. Die erfindungsgemäßen Verbundschichten sind nicht nur gegen Heißluft und Lösungsmittel, wie Alkohol, Aceton, Essigsäureethylester und verdünnte Laugen und Säuren resistent, sondern übersteht auch das Kochen in Wasser. Neben der sehr guten Haftung der Konditionierungsschicht auf Metall, ist die Schicht äußerst hydrolysebeständig und verhindert den Wasserzutritt zur Metalloberfläche und damit die Metallkorrosion.

## Patentansprüche

1. Oberflächenkonditionierter Abformlöffel für die Dentaltechnik, dadurch gekennzeichnet, daß er zumindest im Bereich der Aufnahme des Abformmaterials mit einer dauerhaften Verbundschicht versehen ist, die eine definierte Haftfestigkeit zu Abformmaterialien über mehrere Abformungen gewährleistet.

2. Oberflächenkonditionierter Abformlöffel nach Anspruch 1., dadurch gekennzeichnet, daß er zumindest im Bereich der Aufnahme des Abformmaterials mit einer Verbundschicht versehen ist, die aus einer verfestigten Phenolharz-Methacrylat-Dispersion besteht.

3. Oberflächenkonditionierter Abformlöffel nach Anspruch 1. und 2. oder 3., dadurch gekennzeichnet, daß einem metallischen Abformlöffel eine, zumindest im Bereich der Aufnahme des Abformmaterials, gesandstrahlte Oberfläche gegeben ist, die mit einer silikatischen Schicht überzogen ist und darauf eine verfestigte Phenolharz-Methacrylat-Dispersion aufgebracht ist.

4. Dispersion zur Herstellung einer Verbundschicht nach Anspruch 1. und 2. oder 3., dadurch gekennzeichnet, daß einer Phenolharz-Dispersion, die zumindest hydroxymethyliertes Phenol mit freien Methylolgruppen, dispergiertes Acrylat, dest. Wasser und Aceton enthält, eine oder mehrere olefinische ungestättigte ein- bzw. mehrfunktionelle Methacrylatverbindungen zugesetzt sind.

5. Dispersion zur Herstellung einer Verbundschicht nach Anspruch 1. und 2. oder 3., dadurch gekennzeichnet, daß 100 ml einer Phenolharzacrylat-Dispersion, die zumindest 0,5 bis 5 g hydroxymethyliertes Phenol mit freien Methylolgruppen, 1 bis 5 g dispergiertes Acrylat, 10 bis 30 ml dest. Wasser, 10 bis 30 ml Aceton enthält, 2 bis 20 g einer oder mehrerer olefinischer ungestättigter ein- bzw. mehrfunktioneller Methacrylatverbindungen zugesetzt sind.

6. Dispersion nach Anspruch 4. oder 5., dadurch gekennzeichnet, daß es sich bei den Methacrylatverbindungen um Urethanmethacrylate und/oder Hydroximethacrylate bzw. andere mono- und mehrfunktionelle Methacrylate handelt.

7. Dispersion nach Anspruch 4. oder 5., dadurch gekennzeichnet, daß der Dispersion 1 bis 10 g eines funktionellen Alkoxysilans zugesetzt sind.

8. Disperion nach Anspruch 7., dadurch gekennzeichnet, daß es sich bei den Alkoxysilanen um solche mit drei, zwei oder einer Meth- bzw. Ethoxygruppe und mindestens einer funktionellen organischen Gruppe handelt.

9. Dispersion nach Anspruch 8., dadurch gekennzeichnet, daß es sich bei der funktionellen organischen Gruppe des Alkoxysilans um eine Alkyl-, Phenyl-, Vinyl-, Methacrylat- oder Epoxygruppe handelt.

10. Dispersion nach Anspruch 7. bis 9., dadurch gekennzeichnet, daß es sich bei den Alkoxysilanen um Vinyltrimethoxysilan, Methacrylsäure-3-trimethoxysilan, 3-Glycidoxypropyltriethoxysilan oder Aminoethylaminopropyltrimethoxysilan handelt.

11. Dispersion nach einem der vorstehenden Ansprüche, dadurch gekennzeichnet, daß die Dispersion einen pH-Wert zwischen 7,2 und 8 aufweist.

12. Oberflächenkonditionierter Abformlöffel nach Anspruch 1., dadurch gekennzeichnet, daß er zumindest im Bereich der Aufnahme des Abformmaterials gesandstrahlt ist.

13. Oberflächenkonditionierter Abformlöffel nach Anspruch 12., dadurch gekennzeichnet, daß die gesandstrahlte Oberfläche mit einer Silikatschicht versehen ist.

14. Oberflächenkonditionierter Abformlöffel nach Anspruch 13., dadurch gekennzeichnet, daß auf der Silikatschicht eine Silikonölschicht eingebrannt ist.

15. Verfahren zur Herstellung einer haftfesten, wasserundurchlässigen und hydrolysebeständigen Verbundschicht für Abformlöffel der Dentaltechnik, dadurch gekennzeichnet, daß zumindest auf den Bereich des Abformlöffels, der der Aufnahme des Abformmaterials dient eine Phenolharz-Methacrylat-Dispersion aufgebracht wird und diese einer Temperaturbehandlung im Bereich von 120 bis 220°C unterworfen wird.

16. Verfahren zur Herstellung einer haftfesten, wasserundurchlässigen und hydrolysebeständigen Verbundschicht für Abformlöffel der Dentaltechnik nach Anspruch 15, dadurch gekennzeichnet, daß der Abformlöffel vor Auftrag einer Phenolharz-Methacrylat-Dispersion gesandstrahlt und mit einer Silikatschicht versehen wird.

17. Verfahren zur Herstellung eines oberflächenkonditionierten Abformlöffels nach Anspruch 12. bis 14. dadurch gekennzeichnet, daß auf die Metalloberfläche eines Abformlöffels eine silikatische Schicht aufgebracht wird, die mit einer Silkonöl enthaltenden Lösung bestrichen wird, welche einer Temperaturbehandlung unterworfen wird.

18. Verfahren nach Anspruch 16. , dadurch gekennzeichnet, daß die silikatische Schicht mit einer Silikonöl-Essigsäureethylester-Lösung, mit einem Silikonölanteil von 5 bis 50% bestrichen wird, welche einer Temperaturbehandlung zwischen 180 bis 240°C unterworfen wird.
